(19) Europäisches Patentamt
European Patent Office
Office européen des brevets

(11) EP 2 935 197 B1

(12) **EUROPÄISCHE PATENTSCHRIFT**

(45) Veröffentlichungstag und Bekanntmachung des Hinweises auf die Patenterteilung:
**12.07.2017 Patentblatt 2017/28**

(21) Anmeldenummer: **13815709.4**

(22) Anmeldetag: **17.12.2013**

(51) Int Cl.:
***C07C 68/02*** *(2006.01)*

(86) Internationale Anmeldenummer:
**PCT/EP2013/076787**

(87) Internationale Veröffentlichungsnummer:
**WO 2014/095776 (26.06.2014 Gazette 2014/26)**

# (54) VERFAHREN ZUR HERSTELLUNG VON DIARYLCARBONAT

METHOD FOR MANUFACTURING DIARYL CARBONATE

PROCÉDÉ DE FABRICATION DE CARBONATES DE DIARYLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.12.2012 EP 12197656**

(43) Veröffentlichungstag der Anmeldung:
**28.10.2015 Patentblatt 2015/44**

(73) Patentinhaber: **Covestro Deutschland AG**
**51373 Leverkusen (DE)**

(72) Erfinder:
- **KÖHLER, Karl-Heinz**
  **52078 Aachen-Brand (DE)**
- **KAHNIS, Henning**
  **50667 Köln (DE)**
- **VANDEN EYNDE, Johan**
  **B-9052 Zwijnaarde (BE)**
- **DENECKER, Gabriel**
  **B-2920 Kalmthout (BE)**
- **LEIBERICH, Ricarda**
  **63263 Neu-Isenburg (DE)**
- **HALLENBERGER, Kaspar**
  **51375 Leverkusen (DE)**
- **KRÄMER, Korbinian**
  **50733 Köln (DE)**
- **LIPSKI, Florian**
  **40235 Düsseldorf (DE)**

(74) Vertreter: **Levpat**
**c/o Covestro AG**
**Gebäude 4825**
**51365 Leverkusen (DE)**

(56) Entgegenhaltungen:
**EP-A1- 2 371 806 US-A- 5 424 473**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 935 197 B1

## Beschreibung

Die vorliegende Anmeldung betrifft ein Verfahren zur kontinuierlichen Herstellung von Diarylcarbonat aus Phosgen und wenigstens einer Monohydroxyverbindung (Monophenol) in Gegenwart von Katalysatoren, sowie deren Verwendung zur Herstellung von Polycarbonaten.

Es ist bekannt, Diarylcarbonat durch Umsetzung einer aromatischen Hydroxyverbindung (Monophenol) mit einem halogenierten Carbonyl herzustellen. Beispielsweise sind Verfahren zur Herstellung eines Diarylcarbonats durch Reaktion einer aromatischen Hydroxyverbindung mit Phosgen in Anwesenheit eines Lösungsmittels und Natriumhydroxid als Katalysator allgemein bekannt. Dabei wirkt sich die Verwendung von Lösungsmitteln und Natronlauge nachteilig aus, da durch die wässrige Lauge eine teilweise Verseifung von Phosgen oder Chlorkohlensäureester stattfinden kann, große Mengen Kochsalz als Nebenprodukt anfallen und das Lösungsmittel und Katalysator zurückgewonnen werden müssen.

Aus diesem Grunde ist ein Verfahren zur Herstellung von Diarylcarbonat in Abwesenheit von Lösungsmittel von besonderem Interesse. Da kein Lösungsmittel verwendet wird, muss dieses auch nicht recycelt werden. Wird eine aromatische Hydroxyverbindung zudem direkt mit Phosgen umgesetzt, entsteht Chlorwasserstoff (HCl) als Nebenprodukt anstatt von Natriumchlorid. Demnach kann das Recycling des Nebenproduktes (HCl) vereinfacht werden, da hier ein Ausschleusen der Halogenide deutlich reduziert wird, und die Abwasseraufbereitung deutlich vereinfacht wird.

Die Herstellung von Diarylcarbonaten und insbesondere Diphenylcarbonat durch Reaktion von Monophenolen und Phosgen ohne Alkali und ohne Verwendung von Lösungsmitteln in Gegenwart von einem Katalysator durch den Direktphosgenierungsprozess sind untersucht worden und prinzipiell in der Literatur beschrieben. Siehe hierzu Fig. 1.

Bei der Reaktion beispielsweise von Phenol mit Phosgen wird zunächst im ersten Reaktionsschritt das Zwischenprodukt Chlorameisensäurephenylester (CASPE) und HCl gebildet. CASPE reagiert anschließend mit einem weiteren Phenolmolekül zum DPC und einem weiteren HCl Molekül.

Vorschläge für Verfahren ohne Lösungsmittel mit löslichen Katalysatoren werden in US 2,837,555, US 3,234,263 und US 2,362,865 beschrieben.

In dieser Hinsicht gibt es daher Vorschläge, eine direkte Umsetzung von aromatischen Hydroxyverbindungen mit einem halogenierten Carbonyl in der Gasphase durchzuführen. Dies hat jedoch Nachteile, da diese Reaktionen meistens bei hohen Temperaturen und/oder im Teilvakuum betrieben werden.

Eine weitere Herangehensweise ist eine binäre Gas-Flüssigphase-Reaktion, wie in DE 2447348 C2 offenbart. In diesem Fall wird in der Regel die aromatische Hydroxyverbindung als Flüssigphase verwendet. Um die Effizienz möglichst zu steigern, muss das halogenierte Carbonyl möglichst komplett umgesetzt werden. Um dies zu erreichen, wird die aromatische Hydroxyverbindung im Überschuss zum halogenierten Carbonyl verwendet. Dies bedeutet jedoch, dass ein erhöhter Recyclingbedarf für die aromatische Hydroxyverbindung besteht.

Aus EP 2 371 806 A1 ist ein Verfahren zur kontinuierlichen Herstellung von Diarylcarbonaten aus Phosgen und wenigstens einer Monohydroxyverbindung (Monophenol) in Gegenwart von Katalysatoren bekannt, sowie deren Verwendung zur Herstellung von Polycarbonaten. Der dabei anfallende Chlorwasserstoff wird durch elektrochemische Oxidation unter Verwendung einer Gasdiffusionselektrode als Sauerstoffverzehrkathode zu Chlor umgesetzt, wobei das Chlor zur Herstellung des Phosgens rückgeführt wird.

Daher ist eine Verwendung der aromatischen Hydroxyverbindung mit dem halogenierten Carbonyl in fast äquimolaren Mengen deutlich bevorzugt. Jedoch ist bei einem fast äquimolaren Einsatz das Gleichgewicht ungünstig beeinflusst, weswegen der Umsatz gemäß bekannten Methoden unzureichend ist und daher das Verfahren wenig wirtschaftlich. Zudem kann das Wasserstoffchlorid, das als Nebenprodukt während der Umsetzung entsteht, die Konzentration an halogeniertem Carbonyl deutlich reduzieren und hat daher ebenfalls einen negativen Einfluss auf die Umsetzung.

Angesichts der oben genannten Nachteile besteht der Bedarf nach einer verbesserten Verfahrensführung zur Herstellung von Diarylcarbonat.

Ausgehend vom oben geschilderten Stand der Technik bestand die Aufgabe der vorliegenden Erfindung darin, ein Diarylcarbonat-Herstellungsverfahren bereitzustellen, welches Produkte in hoher Reinheit und guter Ausbeute liefert und eine vereinfachte Rückführung von Nebenprodukten, die aus der Polycarbonat-Produktion stammen, ermöglicht.

Wie bereits beschrieben, besteht jedoch das Problem, dass bei einer direkten Reaktion einer aromatischen Hydroxyverbindung mit einem halogenierten Carbonyl in der Gasphase oder Gas-Flüssigphase größere Mengen an Gas entstehen (beispielsweise Phosgen oder Wasserstoffchlorid).

Die vorliegende Erfindung bietet eine Lösung zu den oben genannten Problemen.

Weiterhin ist eine zu lösende Aufgabe, die Phosgenlöslichkeit zu erhöhen und damit einen flüssigen Eintritt in den Reaktor zu ermöglichen. Durch die aus der erhöhten Phosgenlöslichkeit resultierende höhere Phosgenkonzentration in der Flüssigphase ergibt sich ein verbesserter Phosgenumsatz.

Die vorliegende Erfindung betrifft demnach ein Verfahren zur Herstellung eines Diarylcarbonats, das die vorgehend genannten Nachteile nicht aufweist.

Gegenstand der vorliegenden Erfindung ist demnach ein Verfahren zur Herstellung von Diarylcarbonaten ausgehend von Monophenolen und Carbonyldihalogenid, das dadurch gekennzeichnet ist, dass die genannte Reaktion unter Druck stattfindet und daher das Phosgen in großer Konzentration in der Flüssigphase vorliegt. Dabei wird die Phosgenlöslichkeit durch den erhöhten Druck signifikant erhöht, im Gegensatz zur HCl-Löslichkeit die nur unwesentlich steigt. Demnach wir das chemische Gleichgewicht durch den erhöhten Druck nicht negativ beeinflusst, im Gegensatz zu höheren G/A Verhältnissen.

Durch die Umsetzung einer aromatischen Hydroxyverbindung und eines halogenierten Carbonyls unter Druck konnten daher die o.g. Probleme überraschenderweise gelöst werden wenn folgende Gleichung eingehalten wird:

$$G/A < 0{,}010\ [m/sec],$$

wobei G den Eintritts-Volumenstrom des flüssigen oder gelösten halogenierten Carbonyls, das in den Reaktor geleitet wird, in [$m^3$/sec] und A die innere Querschnittsfläche orthogonal zur Längsachse in [$m^2$] darstellen, und wobei das Verhältnis der beiden Größen auch als Leerrohrgeschwindigkeit bezeichnet wird.

Besonders bevorzugt ist G/A < 0,0095 [m/sec], ganz besonders bevorzugt < 0,0090 [m/sec], insbesondere bevorzugt 0,0075 [m/sec], bevorzugt aber wenigstens 0,0005 [m/sec].

Der verbesserte Phosgenumsatz wurde in Tab. 2 und Fig. 5 durch die graphische Darstellung der Phosgenkonzentration im Reaktoraustritt abgebildet. Es ist zu erkennen, dass gerade unterhalb eines G/A Verhältnisses von 0,010 [m/sec], insbesondere unterhalb vom 0,009 [m/sec] der Phosgenumsatz nochmal exponentiell steigt, was nicht zu erwarten war.

Bevorzugt sind die verschiedenen Komponenten im System mehrheitlich in flüssiger Form. Zudem wird folgende Gleichung als vorteilhaft angesehen:

$$H/D \geq 2$$

wobei H für einen beispielhaft betrachteten, aufrecht stehenden, zylindrischen Reaktor (Reaktionskolonne, Blasensäule) die Höhe der Flüssigphase im Reaktor im gasfreien Zustand von der Reaktorunterseite bis zum oberen Rand der flüssigen Phase bedeutet, und D der Durchmesser des Reaktors ist.

Besonders bevorzugt werden sowohl die aromatische Hydroxyverbindung als auch das halogenierte Carbonyl in flüssiger Phase in den Reaktor geleitet.

Bevorzugt werden die aromatische Hydroxyverbindung und das halogenierte Carbonyl am unteren Ende des Reaktors eingeführt.

Die bevorzugte Ausführungsform des Reaktors hat die Gestalt einer Blasensäule. Der Reaktor in Form einer Blasensäule ist gekennzeichnet durch ein Verhältnis von Höhe H der Flüssigphase im Reaktor im gasfreien Zustand von der Reaktorunterseite bis zum oberen Rand der flüssigen Phase zu Durchmesser D H/D = 2-80, bevorzugt 2-50, besonders bevorzugt 6-20. Der Zulauf der vorgemischten Reaktanden zum Reaktor erfolgt bevorzugt flüssig am unteren Ende der Blasensäule, wodurch ein aufwärtsgerichteter Strom durch den Reaktor entsteht. Der erfindungsgemäße Blasensäulenreaktor wird eingesetzt für Reaktionen mit Gasentwicklung. Durch eingeschränkte Löslichkeit des durch die Reaktion entstehenden Gases bildet sich über die Reaktorlänge eine Gasphase. Bevorzugt verfügt der eingesetzte Blasensäulenreaktor im oberen Teil über einen Überlauf, durch den die Flüssigphase von der Gasphase getrennt wird. Die Gasphase wird am Kopf des Reaktors abgeführt. Dabei wird bevorzugt das Verhältnis zwischen Abstand der Mitte des Überlaufrohrs zum Kopf des Reaktors L und dem Reaktordurchmesser D von L/D ≥ 1 eingehalten. Andere Konstruktionen wie z. B. ringförmige Überläufe oder Erweiterungen des Kopfdurchmessers zur Reduzierung des Tropfenmitrisses in der Gasphase sind dem Stand der Technik genauso zu entnehmen wie auch Blasenabscheider, die dem genannten Überlaufrohr nachgeschaltet werden können, um Gasmitriss in der Flüssigkeit zu vermeiden. Der Reaktor kann als reine Blasensäule oder mit Einbauten wie Schüttungen, strukturierten Packungen, oder bevorzugt segmentierenden Einbauten wie beispielsweise perforierten Lochblechen ausgeführt sein. Die Wahl der Lochblechgeometrien (Lochdurchmesser, Lochanzahl und daraus resultierender Anteil der freien Fläche der Lochbleche) und der Anzahl der Segmente, in die der Reaktor unterteilt wird, erfolgt nach den üblichen Regeln der Technik dergestalt, dass die Verweilzeitverteilung der Flüssigkeit möglichst der eines Reaktors mit Pfropfenströmung entspricht und gleichzeitig ein Anstauen des Gases unter den Lochböden möglichst reduziert wird, um das effektive Flüssigkeitsvolumen und damit die Flüssigkeitsverweilzeit im Reaktor möglichst groß zu halten. Insbesondere kann es dazu auch angeraten sein, über die Höhe des Reaktors entsprechend der lokalen Gasentstehung infolge der ablaufenden Reaktion unterschiedliche Lochblechgeometrien einzusetzen.

Der oben beschriebene Blasensäulenreaktor wird eingesetzt für die Direktphosgenierung von aromatischen

Hydroxyverbindungen, bevorzugt für die Direktphosgenierung von Phenol zu Diphenylcarbonat und HCl. Die Reaktion wird bevorzugt unter Drücken von 3-100bar gefahren, besonders bevorzugt 12-25bar, und bei Temperaturen von bevorzugt 120-220°C, besonders bevorzugt 170-200°C. Vorzugsweise findet die Reaktion im Blasensäulenreaktor in Abwesenheit eines Lösungsmittels statt, besonders bevorzugt in der Schmelze statt.

Für die Reaktion wird bevorzugt als homogener Katalysator Pyridin bzw. Pyridin*HCl und besonders bevorzugt $TiCl_4$ bzw. $Ti(OPh)_4$ oder $AlCl_3$ eingesetzt. Die Flüssigkeitsverweilzeit beträgt bevorzugt 0,5-4h, besonders bevorzugt 0,5-2h. Alternativ kann die Blasensäule auch in Form einer Kaskade von Blasensäulen aufgeteilt werden, wobei sich die Verweilzeit pro Blasensäule dementsprechend reduziert. Die Mischung aus Phenol, flüssigem oder gelöstem Phosgen und dem eingesetzten homogenen Katalysator wird in einer geeigneten Mischdüse zusammengeführt und dem Reaktor von unten in aufwärtsgerichteter Strömung zugeführt. Die Mischung der genannten Komponenten kann aber auch erst im Reaktor selbst erfolgen, dazu kann der Reaktor beispielsweise im unteren Eintrittsbereich mit nach dem Stand der Technik bekannten statischen oder aktiven Mischelementen ausgestattet sein. Der Reaktor kann für die bevorzugte adiabate Betriebsweise in geeigneter Weise isoliert sein.

Durch die während der Reaktion entstehende und nur eingeschränkt im Reaktionsgemisch lösliche Gasphase bildet sich im Reaktor bevorzugt ein homogenes Strömungsregime gemäß Fig. 2 aus. Das Reaktordesign wird in der Weise durchgeführt, so dass sich bevorzugt eine Gas-Leerrohrgeschwindigkeit am Reaktoraustritt von 1-5 cm/s, besonders bevorzugt von 2-3 cm/s ergibt, so dass nur sehr eingeschränkte Rückvermischung erfolgt. Der Gesamtanteil der Gasphase im Blasensäulenreaktor beträgt erfindungsgemäß bevorzugt zwischen 10 und 20%. Alternativ kann der Reaktor auch im heterogenen Strömungsregime bei Gasgeschwindigkeiten oberhalb der genannten Werte betrieben werden; dann kann es zur Wahrung der Flüssigkeits-Verweilzeitverteilung gemäß einer Pfropfenströmung vorteilhaft sein, den Reaktor in oben erläuterter Weise mit segmentierenden Einbauten wie beispielsweise Lochblechen auszustatten.

Der Reaktor wird in der Weise betrieben, dass das molare Verhältnis von Phenol zu Phosgen am Reaktoreintritt zwischen 5:1 und 2:1 beträgt, besonders bevorzugt zwischen 3:1 und 2,2:1. Am Reaktoraustritt beträgt der Phosgengehalt bevorzugt <1%, besonders bevorzugt <10ppm und der CASPE-Gehalt bevorzugt <1%, besonders bevorzugt <100ppm. Vergleiche Fig. 4.

Der Blasensäulenreaktor wird beim durchzuführenden Verfahren der Direktphosgenierung von Phenol zu Diphenylcarbonat und HCl in einer Stufe, bevorzugt in kaskadierter Bauweise mit zwei Stufen eingesetzt. Alternativ können auch mehr als zwei Stufen in kaskadierter Bauweise verwendet werden. Im bevorzugten Fall wird die erste Reaktionsstufe unter Drücken von 3-100bar, besonders bevorzugt 12-25bar bei Temperaturen von bevorzugt 120-220°C, besonders bevorzugt 170-200°C betrieben. Die Verweilzeit in der ersten Stufe beträgt bevorzugt 0,5-4h, besonders bevorzugt 0,5-2h. Die Mischung aus Phenol, flüssigem Phosgen und dem eingesetzten homogenen Katalysator wird in einer geeigneten Mischdüse zusammengeführt und dem Reaktor von unten in aufwärtsgerichteter Strömung zugeführt. Der Reaktor kann für die bevorzugte adiabate Betriebsweise in geeigneter Weise isoliert sein.

Der Reaktor wird in der Weise betrieben, dass das molare Verhältnis Phenol zu Phosgen am Reaktoreintritt zwischen 5:1 und 2:1 beträgt, besonders bevorzugt zwischen 3:1 und 2,2:1. Am Reaktoraustritt beträgt der Phosgengehalt in der Flüssigphase bevorzugt <1%, besonders bevorzugt <200ppm und insbesondere <100ppm. Vergleiche Fig. 4. In der Gasphase beträgt der Phosgengehalt bevorzugt <1%, besonders bevorzugt <2000ppm.

Die zweite Reaktionsstufe wird im bevorzugten Fall unter Drücken von 3-100bar, besonders bevorzugt 12-25bar bei Temperaturen von bevorzugt 120-220°C, besonders bevorzugt 200°-210°C betrieben. Die Verweilzeit beträgt bevorzugt 0,2-4h, besonders bevorzugt 0,2-2h. Alternativ kann die Blasensäule auch in Form einer Kaskade von Blasensäulen aufgeteilt werden, wobei sich die Verweilzeit pro Blasensäule dementsprechend reduziert. Die aus der ersten Reaktionsstufe stammende Mischung aus hauptsächlich Phenol, DPC und CASPE und dem eingesetzten homogenen Katalysator wird auf die Reaktionstemperatur vorgeheizt und der zweiten Reaktionsstufe von unten in aufwärtsgerichteter Strömung zugeführt. Der Reaktor kann für die bevorzugte adiabate Betriebsweise in geeigneter Weise isoliert sein.

Durch die während der Reaktion entstehende und nur eingeschränkt im Reaktionsgemisch lösliche Gasphase bildet sich im Reaktor bevorzugt ein homogenes Strömungsregime gemäß Fig. 2 aus. Das Reaktordesign wird in der Weise durchgeführt, dass sich bevorzugt eine Gas-Leerrohrgeschwindigkeit am Reaktoraustritt von 1-5 cm/s, besonders bevorzugt von 2-3 cm/s ergibt, so dass nur sehr eingeschränkte Rückvermischung erfolgt. Der Gesamtanteil der Gasphase im Blasensäulenreaktor beträgt erfindungsgemäß zwischen 5 und 35%.

Der Reaktor wird in der Weise betrieben, dass der Phosgengehalt in der Flüssigphase am Reaktoraustritt der zweiten Stufe bevorzugt <100ppm beträgt, besonders bevorzugt <10ppm und der CASPE-Gehalt bevorzugt <2000ppm beträgt, besonders bevorzugt < 100ppm. In der Gasphase beträgt der Phosgengehalt bevorzugt <25ppm.

Die entstehende HCl kann anschließend, falls erforderlich nach geeigneter Reinigung, entweder durch eine Elektrolyse, bevorzugt einer Membranelektrolyse, ggf. mithilfe einer Sauerstoffverzehrkathode, oder eine thermische Oxidation in Anwesenheit von Sauerstoff und eines geeigneten Katalysators (Deacon-Prozess) wieder zu Chlor umgesetzt werden, der wiederrum durch Umsetzung mit Kohlenmonoxid zur Herstellung von Phosgen wiederverwendet werden kann. Dieses Phosgen wird dann bevorzugt zumindest als Teilmenge zur Umsetzung mit einem Monophenol

eingesetzt, um das Diarylcarbonat herzustellen.

Im Rahmen der Erfindung hergestellte Diarylcarbonate sind bevorzugt solche der allgemeinen Formel (I)

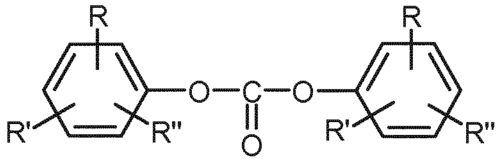

(I)

wobei R, R' und R" unabhängig voneinander H, lineares oder verzweigtes, gegebenenfalls substituiertes C1-C34-Alkyl, bevorzugt C1-C6-Alkyl, besonders bevorzugt C1-C4-Alkyl, C1-C34-Alkoxy, bevorzugt C1-C6-Alkoxy, besonders bevorzugt C1-C4-Alkoxy, C5-C34-Cycloalkyl, C7-C34-Alkylaryl, C6-C34-Aryl oder einen Halogenrest, bevorzugt einen Chlorrest darstellen und R, R' und R" auf beiden Seiten der Formel (I) gleich oder verschieden sein können. R kann auch -COO-R'" bedeuten, wobei R'" H, gegebenenfalls verzweigtes C1-C34 Alkyl, bevorzugt C1-C6-Alkyl, besonders bevorzugt C1-C4-Alkyl, C1-C34-Alkoxy, bevorzugt C1-C6-Alkoxy, besonders bevorzugt C1-C4-Alkoxy, C5-C34-Cycloalkyl, C7-C34-Alkylaryl oder C6-C34-Aryl sein kann. Bevorzugt sind R, R' und R" auf beiden Seiten der Formel (I) gleich. Ganz besonders bevorzugt stehen R, R' und R" für H.

Diarylcarbonate der allgemeinen Formel (I) sind beispielsweise: Diphenylcarbonat, Methylphenylphenyl-carbonate und Di-(methylphenyl)-carbonate, auch als Gemisch, wobei die Stellung der Methylgruppe an den Phenylringen beliebig sein kann, sowie Dimethylphenyl-phenyl-carbonate und Di-(dimethylphenyl)-carbonate, auch als Gemisch, wobei die Stellung der Methylgruppen an den Phenylringen beliebig sein kann, Chlorphenyl-phenyl-carbonate und Di-(chlorphenyl)-carbonate, wobei die Stellung der Methylgruppe an den Phenylringen beliebig sein kann, 4-Ethylphenyl-phenyl-carbonat, Di-(4-ethylphenyl)-carbonat, 4-n-Propylphenyl-phenyl-carbonat, Di-(4-n-propylphenyl)-carbonat, 4-iso-Propylphenyl-phenyl-carbonat, Di-(4-iso-propylphenyl)-carbonat, 4-n-Butylphenyl-phenyl-carbonat, Di-(4-n-butylphenyl)-carbonat, 4-iso-Butylphenylphenylcarbonat, Di-(4-iso-butylphenyl)-carbonat, 4-tert-Butylphenyl-phenyl-carbonat, Di-(4-tert-butylphenyl)-carbonat, 4-n-Pentylphenyl-phenyl-carbonat, Di-(4-n-pentylphenyl)-carbonat, 4-n-Hexylphenyl-phenyl-carbonat, Di-(4-n-hexylphenyl)-carbonat, 4-iso-Octylphenyl-phenyl-carbonat, Di-(4-iso-octylphenyl)-carbonat,4-n-Nonylphenyl-phenyl-carbonat, Di-(4-n-nonylphenyl)-carbonat, 4-Cyclohexylphenyl-phenyl-carbonat, Di-(4-cyclohexylphenyl)-carbonat, 4-(1-Methyl-1-phenylethyl)-phenyl-phenyl-carbonat, Di-[4-(1-methyl-1-phenylethyl)-phenyl]-carbonat, Biphenyl-4-yl-phenylcarbonat, Di-(biphenyl-4-yl)-carbonat, (1-Naphthyl)-phenyl-carbonat, (2-Naphthyl)-phenyl-carbonat, Di-(1-naphthyl)-carbonat, Di-(2-naphthyl)-carbonat, 4-(1-Naphthyl)-phenyl-phenyl-carbonat, 4-(2-Naphthyl)-phenyl-phenyl-carbonat, Di-[4-(1-naphthyl)phenyl]-carbonat, Di-[4-(2-naphthyl)phenyl] -carbonat, 4-Phenoxyphenyl-phenyl-carbonat, Di-(4-phenoxyphenyl)-carbonat, 3-Pentadecylphenyl-phenyl-carbonat, Di-(3-pentadecylphenyl)-carbonat, 4-Tritylphenylphenyl-carbonat, Di-(4-tritylphenyl)-carbonat, Methylsalicylat-phenyl-carbonat, Di-(methylsalicylat)-carbonat, Ethylsalicylat-phenyl-carbonat, Di-(ethylsalicylat)-carbonat, n-Propylsalicylat-phenyl-carbonat, Di-(n-propylsalicylat)-carbonat, iso-Propylsalicylat-phenyl-carbonat, Di-(iso-propylsalicylat)-carbonat, n-Butylsalicylat-phenyl-carbonat, Di-(n-butylsalicylat)-carbonat, iso-Butylsalicylat-phenyl-carbonat, Di-(iso-butylsalicylat)-carbonat, tert-Butylsalicylat-phenyl-carbonat, Di-(tert-butylsalicylat)-carbonat, Di-(phenylsalicylat)-carbonat und Di-(benzylsalicylat)-carbonat.

Bevorzugte Diarylcarbonate sind: Diphenylcarbonat, 4-tert-Butylphenyl-phenyl-carbonat, Di-(4-tert-butylphenyl)-carbonat, Biphenyl-4-yl-phenyl-carbonat, Di-(biphenyl-4-yl)-carbonat, 4-(1-Methyl-1-phenylethyl)-phenyl-phenyl-carbonat und Di-[4-(1-methyl-1-phenylethyl)-phenyl]-carbonat.

Besonders bevorzugt ist Diphenylcarbonat.

Im Rahmen der Erfindung geeignete aromatische Hydroxyverbindungen sind bevorzugt solche der allgemeinen Formel (III)

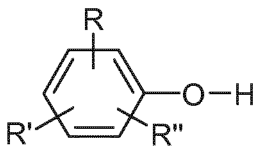

(III)

worin R, R' und R" unabhängig voneinander die für die allgemeine Formel (I) genannte Bedeutung haben können.

Solche aromatischen Hydroxyverbindungen sind beispielsweise: Phenol, o-, m- oder p-Kresol, auch als Gemisch der Kresole, Dimethylphenol, auch als Gemisch, wobei die Stellung der Methylgruppen am Phenolring beliebig sein kann, z.B. 2,4-, 2,6-, oder 3,4-Dimethylphenol, o-, m-oder p-Chlorphenol, o-, m- oder p-Ethylphenol, o-, m- oder p-n-Propylphenol, 4-iso-Propylphenol, 4-n-Butylphenol, 4-iso-Butylphenol, 4-tert-Butylphenol, 4-n-Pentylphenol, 4-n-Hexylphenol, 4-isoOctylphenol, 4-n-Nonylphenol, o-, m- oder p-Methoxyphenol, 4-Cyclohexylphenol, 4-(1-Methyl-1-phenylethyl)-phenol, Biphenyl-4-ol, 1-Naphthol, 2-1-Naphthol, 4-(1-Naphthyl)phenol, 4-(2-Naphthyl)-phenol, 4-Phenoxyphenol, 3-Pentadecylphenol, 4-Tritylphenol, Methylsalicylsäure, Ethylsalicylsäure, n-Propylsalicylsäuret, iso-Propylsalicylsäure, n-Butylsalicylsäure, iso-Butylsalicylsäure, tert-Butylsalicylsäure, Phenylsalicylsäure und Benzylsalicylsäure.

Bevorzugte aromatischen Hydroxyverbindungen sind Phenol, 4-tert-Butylphenol, Biphenyl-4-ol und 4-(1-Methyl-1-phenylethyl)-phenol.

Besonders bevorzugt ist Phenol.

Für die in der ersten Reaktionskolonne auftretenden Reaktionsschritte können aus der Literatur bekannte Umesterungskatalysatoren eingesetzt werden. Dies sind aus der Literatur bekannte Umesterungskatalysatoren für die Dialkylcarbonat-Phenol-Umesterung, wie z. B. $AlX_3$, $TiX_3$, $UX_4$, $TiX_4$, $VOX_3$, $VX_5$, $ZnX_2$, $FeX_3$, $PbX_2$ und $SnX_4$, worin X für Halogen-, Acetoxy-, Alkoxy- oder Aryloxyreste steht (DE-OS 2 58 412). Besonders bevorzugte einsetzbare Katalysatoren sind Metallverbindungen wie $AlX_3$, $TiX_4$, $PbX_2$ und $SnX_4$, wie beispielsweise Titantetrachlorid, Titantetramethoxid Titantetraphenoxid, Titantetraethoxid, Titantetraisopropylat, Titantetradodecylat, Zinntetraisooctylat und Aluminiumtriisopropylat. Ganz besonders bevorzugt sind Metallverbindungen $TiX_4$, insbesondere $TiCl_4$. Die genannten Metallverbindungen werden bevorzugt in Mengen von 0,001 bis 5 Gew.-%, bevorzugt von 0,005 bis 5 Gew.-% und besonders bevorzugt von 0,01 bis 5 Gew.-%, bezogen auf das Gewicht des umzusetzenden Reaktionsgemischs, eingesetzt. Bevorzugt sind Aluminiumoxid, Titanoxid, Zirkonoxid, Calciumtitanat, Magnesiumtitanat und Magnesiumaluminiumhydrotalcit, die im neuen Verfahren vorzugsweise als Festbett, in einer Kolonne mit Katalysator als Füllkörper oder Beschichtung auf strukturierten Packungen (in einer Blasensäule auch dreiphasiger Betrieb mit suspendiertem Katalaysator denkbar (Slurry-Blasensäule, Flüssig-Wirbelschicht)), eingesetzt werden, und Titanchlorid, Zirkonchlorid, Aluminiumchlorid und Titanphenolat, die als homogene Katalysatoren eingesetzt werden.

Weitere bevorzugte Katalysatoren sind basische stickstoffhaltige Katalysatoren, insbesondere quaternäre Ammonium-Salze wie beispielsweise organische und anorganische Salze wie Tetramethylammonium- und Tetraethymammonium-Salze. Zudem sind ebenfalls aromatische stickstoffhaltige heterozyklische Basen, wie beispielsweise Pyridin, 2-Methoxypyridin, 2-Hydroxypyridin, Quinolin, Isoquinolin, Picolin, Acridin, Imidazole, 2-Methylimidazol, Benzimidazole, Pyrazol, Pyrazin, Pyrimidin Triazole und Benztriazole, sowie deren Salze einschließlich organische und anorganische Salze des vorangenannten aromatischen stickstoffhaltige heterozyklische Basen. Weiterhin sind Polymere der vorgenannten Verbindungen, wie beispielsweise Polyvinylpyridin ebenfalls geeignete Katalysatoren. Bevorzugt sind Pyridin, $\alpha$-Picolin, $\beta$- und $\gamma$-gemischte Picoline, Insoquinolin, 2-Hydroxypyridin und Imidazol. Besonders bevorzugt ist Pyridin.

Die Menge an stickstoffhaltigem Katalysator liegt bevorzugt im Bereich von 0,1 bis 10 mol%, bevorzugt von 0,5 bis 5 mol%, in Bezug auf das Monophenol. Der stickstoffhaltige Katalysator wird im Reaktionsmedium schnell zu dem entsprechenden Hydrochlorid umgesetzt. Daher wird eine gleiche Katalysatoraktivität erreicht, wenn Chloride der oben genannten Verbindungen verwendet werden. Es können jedoch ebenfalls Bromide, Sulfate und Nitrate verwendet werden, sowie schwache Säuren der oben genannten Verbindungen, wie Formate, Acetate oder Phosphate.

Besonders bevorzugt sind Aluminiumoxid, Aluminiumchlorid und $TiCl_4$ oder Pyridin.

Halogen bedeutet im Rahmen der Erfindung Fluor, Chlor oder Brom, bevorzugt Fluor oder Chlor, besonders bevorzugt Chlor.

Weitere einsetzbare Katalysatoren sind zinnorganische Verbindungen der allgemeinen Formel $(R_{11})_{4}$-X-Sn(Y)X, in der Y für einen Rest $OCOR_{12}$, OH oder OR steht, wobei $R_{12}$ $C_1$-$C_{12}$-Alkyl, $C_6$-$C_{12}$-Aryl oder $C_7$-$C_{13}$-Alkylaryl bedeutet, $R_{11}$ unabhängig von $R_{12}$ die Bedeutung von $R_{12}$ hat und x eine ganze Zahl von 1 bis 3 bedeutet, Dialkylzinnverbindungen mit 1 bis 12 C-Atomen im Alkylrest oder Bis-(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew: % (vgl. EP 879 A, EP 880 A, EP 39 452 A, DE-OS 34 45 555, JP 79/63023), polymere Zinnverbindungen der Formel -[-$RR_{11}$Sn-O-]-, in welcher R und $R_{11}$ unabhängig voneinander die vorangehend für $R_{12}$ genannte Bedeutung haben, beispielsweise Poly[oxy(dibutylstannylen)] Poly[oxy(dioctylstannylen)], Poly[oxy(butylphenyl-stannylen)] und Poly[oxy(diphenylstannylen)] (DE-OS 34 45 552), polymere Hydroxystannoxane der Formel -[-RSn(OH)-O-]-, beispielsweise Poly(ethylhydroxystannoxan), Poly(butylhydroxystannoxan), Poly-(octylhydroxystannoxan), Poly(undecylhydroxystannoxan) und Poly(dodecylhydroxystannoxane) in Mengen von 0,001 bis 20 Gew.-%, bevorzugt von 0,005 bis 5 Gew.-%, bezogen auf Dialkylcarbonat (DE-OS 40 06 520). Weitere einsetzbare Zinnverbindungen sind Sn(II)oxide der allgemeinen Formel

$$X\text{-}R_2Sn\text{-}O\text{-}R_2Sn\text{-}Y,$$

worin X und Y unabhängig voneinander OH, SCN, $OR_{13}$, $OCOR_{13}$ oder Halogen und R Alkyl, Aryl bedeuten soll, worin $R_{13}$ die vorangehend für $R_{12}$ genannte Bedeutung hat (EP 0 338 760).

Als weitere einsetzbare Katalysatoren kommen Bleiverbindungen, gegebenenfalls zusammen mit Triorganophosphanen, einer Chelatverbindung oder einem Alkalimetallhalogenid, beispielsweise $Pb(OH)_2$-$2PbCO_3$, Pb(OCO-$CH_3)_2$, Pb(OCO-$CH_3)_2$ · 2LiCl, Pb(OCO-$CH_3)_2$ • $2PPh_3$ in Mengen von 0,001 bis 1, bevorzugt von 0,005 bis 0,25 Mol pro Mol Dialkylcarbonat (JP 57/176932, JP 01/093580), sowie andere Blei(II)- und Blei(IV)-verbindungen, wie PbO, $PbO_2$, Mennige, Plumbite, und Plumbate (JP 01/093560), Eisen(III)acetat (JP 61/1 72 852), weiterhin Kupfersalze und/oder Metallkomplexe, beispielsweise von Alkali, Zink, Titan und Eisen (JP 89/005588).

Weiterhin sind in den Verfahren heterogene Katalysatorsysteme einsetzbar. Solche sind beispielsweise Mischoxide aus Silizium und Titan, die durch gemeinsame Hydrolyse von Silizium und Titanhalogeniden erhältlich sind (JP 54/125617) oder Titandioxide mit hoher BET-Oberfläche >20 m2/g (DE-OS 40 36 594)).

Weitere geeignete Katalysatoren sind

a) Aktivkohlen mit nach der BET-Methode bestimmten Oberflächen von 200 bis 3000 m2/g, die aus Sägemehl und anderen Holzabfällen, Stroh, Kohlensorten, Nußschalen, Mineralölteeren, Lignin, Polysacchariden, Polyacrylnitril, Knochen, Torf oder Koksprodukte aus Braun- oder Steinkohlen, bevorzugt aus Holz, Cellulose, Lignin, Bitumen- oder Braunkohle, Torf oder Steinkohlenkoks hergestellt wurde.

b) Alumosilikaten, ausgewählt aus der Gruppe der Zeolithe der allgemeinen Formel

$$M_{2/n}O \,.\, x\, SiO_2 .\, Al_2O_3 .\, y\, H_2O$$

in welcher

M für Kationen wie Protonen oder Metallkationen des Periodensystems der Elemente nach Mendelejew steht,
n für die Wertigkeit des Kations steht,
x für das Mol-Verhältnis $SiO_2.Al_2O_3$ steht, wobei
x eine Zahl von 1,0-50,0 sein kann, und
y für eine Zahl von 0 - 9 steht,

oder zeolith-ähnliche Verbindungen wie ALPO's und SAPO's oder Schichtsilikate des Kaolin-, Serpentin-, Montmorillonit- und Bentonit-Typs oder "pillared clays" oder $SiO_2/Al_2O_3$-Fällungskatalysatoren,

c) Aluminiumoxide oder y-Aluminiumoxide mit nach der BET-Methode bestimmten Oberflächen von 2 bis 500 $m^2/g$,

d) Oxid(e) von Metallen der Gruppe IVB des periodischen Systems wie beispielsweise Oxide des Titans, Zirkoniums oder Hafniums mit nach der BET-Methode bestimmten Oberflächen von 2 bis 500 $m^2/g$,

e) Metallate der allgemeinen Formel

$$A_xB_yO_z$$

in welcher

A für ein mono-, di und/oder trivalentes Metallkation steht und
B für ein tri-, tetra-, penta- und/oder hexavalentes Metallkation steht und
x für eine Zahl von 1 bis 4 steht und
y für eine Zahl von 1 oder 2 steht und
z für eine Zahl von 3, 6, 7 oder 9 steht,

f) Hartstoffe mit metallartigen Eigenschaften (Keramikvorprodukte) der allgemeinen Formel

$$A_xB_yC_zD_w$$

in welcher

A für ein Element aus den Gruppen 3 bis 10, 13 und 14 des periodischen Systems der Elemente (IUPAC Notation) steht und
B für ein Element aus den Gruppen 13, 14, 15 und 16 mit Ausnahme von Sauerstoff steht, C für ein Element aus den Gruppen 14 und 15 steht und
D für ein Element aus den Gruppen 14 und 15 steht und

x für eine Zahl von 1 bis 4 steht und
y für eine Zahl von 1 bis 4 steht und
z für eine Zahl von 0 bis 4 steht und
w für eine Zahl von 0 bis 4 steht

wobei A, B, C und D jeweils aus verschiedenen Gruppen oder bei gleicher Gruppe aus verschiedenen Perioden stammen, mit der Maßgabe, dass A verschieden von Aluminium ist, wenn B Kohlenstoff ist und gleichzeitig z und w gleich 0 sind,

g) Mischhydroxide der allgemeinen Formel (III)

$$[M(II)_{1-x}\, M(III)_x\, M(IV)_y\, (OH)_2]\, A^{n-}{}_{z/n} \cdot m\, H_2O \qquad (III),$$

in welcher

M (II) für ein divalentes Metallkation steht und
M (III) für ein trivalentes Metallkation steht und
M(IV) für ein tetravalentes Metallkation steht und
x für eine Zahl von 0,1 bis 0,5 steht und
y für eine Zahl von 0 bis 0,5 steht
z für 1 + y steht und
m für eine Zahl von 0 bis 1 steht
A für ein anorganisches Anion wie $OH^-$, $NO_3^-$, $CO_3^{2-}$, $SO_4^{2-}$, $CrO_4^{2-}$ oder $Cl^-$ steht.
n für 1 bis 2 steht

h) Organophosphorverbindungen, homogen oder gegebenenfalls polymergebunden.

Gegebenenfalls können die homogenen Katalysatoren auf einem inerten Träger aufgebracht werden, wie in JP 695219, WO 91/06526, US 5 424 473 und EP 516 355 beschrieben.

Die Katalysatoren können einzeln oder in Verbindung miteinander verwendet werden.

In einer bevorzugten Ausführungsform wird der Katalysator in Mengen von 0,5 bis 100 Gew.-% bezogen auf die Menge an Monohydroxyverbindung (Monophenol), bei nicht vollkontinuierlicher Fahrweise bzw. mit Belastungen von 0,1 bis 20 g Monohydroxyverbindung (Monophenol) pro g Katalysator pro Stunde bei vollkontinuierlicher Fahrweise, eingesetzt. In einer anderen bevorzugten Ausführungsform, insbesondere wenn der Katalysator $TiCl_4$ oder $Ti(OPh)_4$ ist, kann der Katalysator auch mit Belastungen von über 20 g Monohydroxyverbindung (Monophenol) pro g Katalysator pro Stunde bei vollkontinuierlicher Fahrweise eingesetzt werden.

Der Katalysator wird bevorzugt zusammen mit dem die aromatische(n) Hydroxyverbindung(en) enthaltenden Strom in gelöster oder suspendierter Form in die erste Reaktionskolonne eingebracht. Alternativ kann der Katalysator auch separat beispielsweise in einem dem Alkylalkohol entsprechenden Alkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Im Rahmen der Erfindung verwendete Dialkylcarbonate sind bevorzugt solche der allgemeinen Formel (IV)

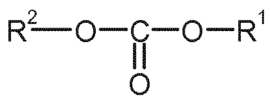

(IV),

wobei R1 und R2 unabhängig voneinander für lineares oder verzweigtes, gegebenenfalls substituiertes C1-C34-Alkyl, bevorzugt für C1-C6-Alkyl, besonders bevorzugt für C1-C4-Alkyl stehen. Dabei können R1 und R2 gleich oder verschieden sein. Bevorzugt sind R1 und R2 gleich.

C1-C4-Alkyl steht im Rahmen der Erfindung beispielsweise für Methyl, Ethyl, n-Propyl, isoPropyl, n-Butyl, sec.-Butyl, tert.-Butyl, C1-C6-Alkyl darüber hinaus beispielsweise fürn-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, C1-C34-Alkyl darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

Die vorangehenden Aufzählungen sind beispielhaft und nicht als Limitierung zu verstehen.

Bevorzugte Dialkylcarbonate sind Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat. Besonders bevorzugt sind Dimethylcarbonat oder Diethylcarbonat. Ganz besonders bevorzugt ist Dimethylcarbonat.

Die Dialkylcarbonate werden bevorzugt aus zyklischen Alkylencarbonaten mit der Formel (V) hergestellt:

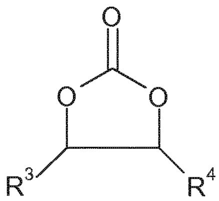

(V),

wobei in der Formel R3 und R4 unabhängig voneinander Wasserstoff, substituiertes oder nicht substituiertes C1-C4-Alkyl, substituiertes oder nicht substituiertes C2-C4-Alkenyl oder substituiertes oder nicht substituiertes C6-C12-Aryl und R3 und R4 gemeinsam mit den beiden Dreiring-C-Atomen einen gesättigten carbozyklischen Ring mit 5 - 8 Ringgliedern bedeuten können.

Die zyklischen Alkylencarbonate werden mit Alkoholen der Formel

R5-OH

umgesetzt, wobei R5 ein geradkettiges oder verzweigtes C1-C4-Alkyl bedeutet.

Als halogeniertes Carbonyl wird bevorzugt Phosgen oder Bromcarbonyl, besonders bevorzugt jedoch Phosgen eingesetzt. Es können ein oder mehrere halogenierte Carbonyle eingesetzt werden.

Das halogenierte Carbonyl kann Verunreinigungen enthalten, insbesondere Anteile an inerten Gasen wie beispielsweise Stickstoff oder Argon, bevorzugt hat das halogenierte Carbonyl jedoch eine Reinheit von wenigstens 90 Gew.-%, bevorzugt wenigstens 95 Gew.-%, besonders bevorzugt wenigstens 99 Gew.-%.

Figur 1: Reaktionsmechanismen

Figur 2: Strömungsregimes in Blasensäulen [Wolf-Dieter Deckwer: Reaktionstechnik in Blasensäulen, Salle + Sauerländer, Frankfurt am Main, 1985, S. 184, Abb. 7.1]

Figur 3: Schema der Blasensäule

Figur 4: Konzentrationsprofile der Flüssigphase im Reaktor

Figur 5: graphische Darstellung des Phosgenumsatzes anhand der Phosgenkonzentration am Reaktoraustritt

## Beispiele

In einen Blasensäulenreaktor mit einer Länge von 14 m wird bei einer Temperatur von 170°C ein Phenol, Katalysator und Phosgen-enthaltender Strom in der Zusammensetzung gemäß Tabelle 1 eingespeist. Die Reaktionsbedingungen werden jeweils so eingestellt, dass verschiedene G/A-Verhältnisse erhalten werden. Dabei wird ermittelt, welche Phosgen-Konzentration am Reaktoraustritt in der Flüssigphase vorhanden ist. Der Verlauf der Konzentrationen (Massenanteile) über die Lauflänge des Reaktors bei einem G/A Verhältnis von 0,00896 m/s ist in Fig. 4 gezeigt. Fig. 5 zeigt den Effekt der deutlichen Verringerung des Phosgengehaltes am Reaktoraustritt in Abhängigkeit des G/A-Verhältnisses. Ab einem G/A-Verhältnis von kleiner als 0,010 nimmt die Verringerung der Phosgenmenge in der Flüssigphase am Reaktoraustritt nochmal deutlich zu.

Tabelle 1

| | | | |
|---|---|---|---|
| T | °C | 170,0 | |
| Gesamtmasse | kg/h | 37141,3 | |
| PHENOL | kg/h \| % | 23756,9 | 64,0 |
| $COCl_2$ | kg/h \| % | 9987,8 | 26,9 |
| DPC | kg/h \| % | 832,3 | 2,2 |
| $Ti(OPh)_4$ | kg/h \| % | 1700,0 | 4,6 |
| SALIPHES | kg/h \| % | 657,2 | 1,8 |
| Gesamtvolumen | m3/h | 37,7 | |

Tabelle 2

| Beispiel | Leerrohrgeschwindigkeit G/A | Phosgenkonzentration Mitte Reaktorlänge | Phosgenkonzentration Reaktor-Austritt |
|---|---|---|---|
| | [m/s] | mg/kg | mg/kg |
| 1* | 0,288 | 1238,7 | 418,7 |
| 2* | 0,164 | 1085,1 | 302,8 |
| 3* | 0,087 | 703,6 | 107,9 |
| 4* | 0,033 | 193,5 | 5,2 |
| 5 | 0,009 | 24,0 | 0,042 |
| 6 | 0,001 | 8,6 | 0,004 |
| *: Vergleichsbeispiel | | | |

**Patentansprüche**

1. Verfahren zur Herstellung von Diarylcarbonat durch Reaktion einer aromatischen Hydroxyverbindung und einem halogenierten Carbonyl in Anwesenheit eines Katalysators in wenigstens einem Blasensäulenreaktor, wobei das Verhältnis G/A kleiner als 0,010 ist, wobei G den Eintritts-Volumenstrom des flüssigen oder gelösten halogenierten Carbonyls in $m^3/s$ und A die innere Querschnittsfläche orthogonal zur Längsachse in $m^2$ darstellt und wobei die Reaktion unter Druck stattfindet.

2. Verfahren gemäß Anspruch 1, wobei die Reaktion in einem Blasensäulenreaktor in Abwesenheit eines Lösungsmittels stattfindet.

3. Verfahren gemäß Anspruch 1 oder 2, wobei G/A im Bereich von 0,0005 [m/s] und 0,0095 [m/s] liegt.

4. Verfahren gemäß einem der Ansprüche 1 bis 3, wobei das Verhältnis H/D größer oder gleich 2 ist und H die Höhe der Flüssigkeitsphase im Reaktor im gasfreien Zustand bedeutet und D der Durchmesser des Reaktors ist.

5. Verfahren gemäß einem der Ansprüche 1 bis 4, wobei die Verweilzeit im Reaktor im Bereich von 0,5 bis 4 Stunden, die Temperatur im Bereich von 120 bis 220 °C und der Druck 3 bis 100 bar beträgt.

6. Verfahren gemäß einem der Ansprüche 1 bis 5, **dadurch gekennzeichnet, dass** als halogeniertes Carbonyl Phosgen verwendet wird.

7. Verfahren gemäß einem der Ansprüche 1 bis 6, **dadurch gekennzeichnet dass** als aromatische Hydroxyverbindung Phenol verwendet wird.

8. Verfahren gemäß einem der Ansprüche 1 bis 7, **dadurch gekennzeichnet, dass** als Katalysator Pyridin bzw. Pyridin*HCl, $TiCl_4$, $Ti(OPh)_4$ oder $AlCl_3$ eingesetzt wird.

9. Verfahren gemäß einem der Ansprüche 1 bis 8, **dadurch gekennzeichnet, dass** die Verfahrensführung in wenigstens zwei Stufen erfolgt.

10. Verfahren nach einem der Ansprüche 1 bis 9, **dadurch gekennzeichnet, dass** der Phosgengehalt am Reaktoraustritt in der Flüssigphase weniger als 100 ppm beträgt.

## Claims

1. Process for preparing diaryl carbonate by reaction of an aromatic hydroxyl compound and a halogenated carbonyl in the presence of a catalyst in at least one bubble column reactor, wherein the ratio G/A is less than 0.010, where G is the entry volume flow rate of the liquid or dissolved halogenated carbonyl in $m^3/s$ and A is the internal cross-sectional area orthogonal to the longitudinal axis in $m^2$ and wherein the reaction takes place under pressure.

2. Process according to Claim 1, wherein the reaction takes place in a bubble column reactor in the absence of a solvent.

3. Process according to Claim 1 or 2, wherein G/A is in the range from 0.0005 [m/s] and 0.0095 [m/s].

4. Process according to any of Claims 1 to 3, wherein the ratio H/D is greater than or equal to 2 and H is the height of the liquid phase in the reactor in the gas-free state and D is the diameter of the reactor.

5. Process according to any of Claims 1 to 4, wherein the residence time in the reactor is in the range from 0.5 to 4 hours, the temperature is in the range from 120 to 220°C and the pressure is 3 to 100 bar.

6. Process according to any of Claims 1 to 5, **characterized in that** the halogenated carbonyl used is phosgene.

7. Process according to any of Claims 1 to 6, **characterized in that** the aromatic hydroxyl compound used is phenol.

8. Process according to any of Claims 1 to 7, **characterized in that** the catalyst used is pyridine or pyridine*HCl, $TiCl_4$, $Ti(OPh)_4$ or $AlCl_3$.

9. Process according to any of Claims 1 to 8, **characterized in that** the process is conducted in at least two stages.

10. Process according to any of Claims 1 to 9, **characterized in that** the phosgene content at the reactor outlet in the liquid phase is less than 100 ppm.

## Revendications

1. Procédé de fabrication de carbonate de diaryle par réaction d'un composé hydroxy aromatique et d'un carbonyle halogéné en présence d'un catalyseur dans au moins un réacteur à colonne à bulles, le rapport G/A étant inférieur à 0,010, G étant le débit volumique d'entrée du carbonyle halogéné liquide ou dissous en $m^3/s$ et A étant la surface de section transversale intérieure orthogonale à l'axe longitudinal en $m^2$, et la réaction ayant lieu sous pression.

2. Procédé selon la revendication 1, dans lequel la réaction a lieu dans un réacteur à colonne à bulles en l'absence d'un solvant.

3. Procédé selon la revendication 1 ou 2, dans lequel G/A se situe dans la plage comprise entre 0,0005 [m/s] et 0,0095 [m/s].

4. Procédé selon l'une quelconque des revendications 1 à 3, dans lequel le rapport H/D est supérieur ou égal à 2, H signifiant la hauteur de la phase liquide dans le réacteur à l'état sans gaz et D étant le diamètre du réacteur.

5. Procédé selon l'une quelconque des revendications 1 à 4, dans lequel le temps de séjour dans le réacteur se situe dans la plage allant de 0,5 à 4 heures, la température dans la plage allant de 120 à 220 °C, et la pression dans la

plage allant de 3 à 100 bar.

6. Procédé selon l'une quelconque des revendications 1 à 5, **caractérisé en ce que** le phosgène est utilisé en tant que carbonyle halogéné.

7. Procédé selon l'une quelconque des revendications 1 à 6, **caractérisé en ce que** le phénol est utilisé en tant que composé hydroxy aromatique.

8. Procédé selon l'une quelconque des revendications 1 à 7, **caractérisé en ce que** la pyridine ou la pyridine*HCl, $TiCl_4$, $Ti(OPh)_4$ ou $AlCl_3$ est utilisé en tant que catalyseur.

9. Procédé selon l'une quelconque des revendications 1 à 8, **caractérisé en ce que** le procédé est réalisé en au moins deux étapes.

10. Procédé selon l'une quelconque des revendications 1 à 9, **caractérisé en ce que** la teneur en phosgène à la sortie du réacteur dans la phase liquide est inférieure à 100 ppm.

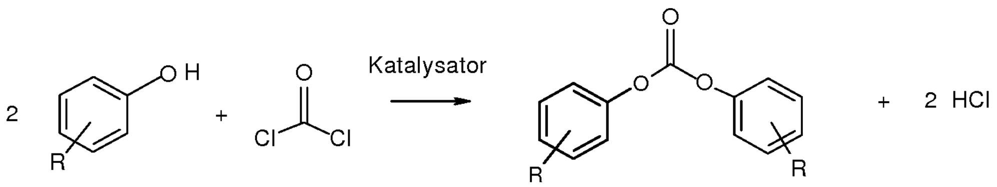
Katalysator

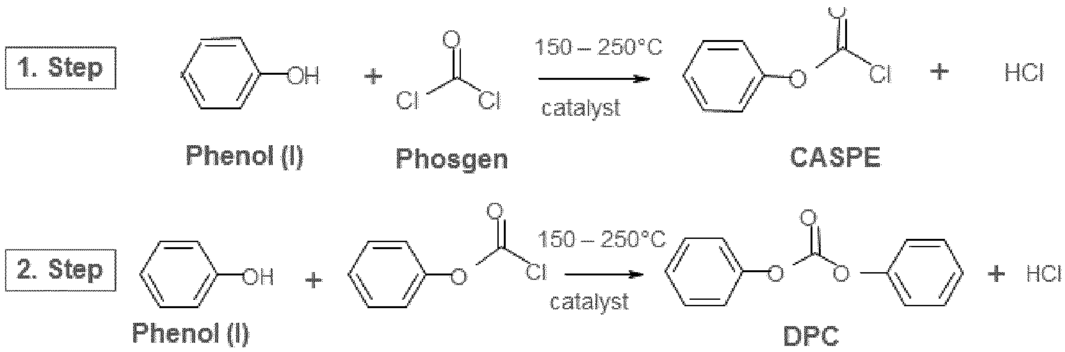
1. Step
150 – 250°C
catalyst
Phenol (I)
Phosgen
CASPE
2. Step
150 – 250°C
catalyst
Phenol (I)
DPC

Fig. 1

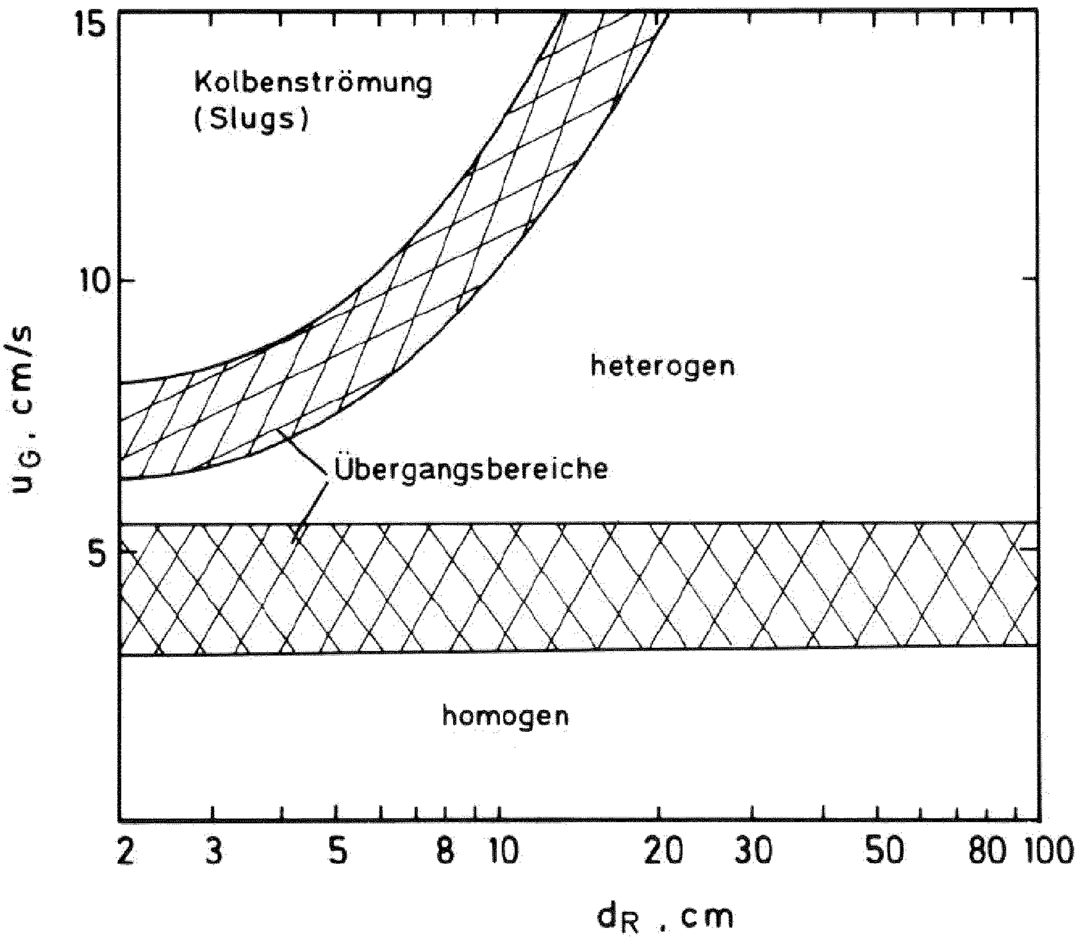
15
10
5
$u_G$, cm/s
Kolbenströmung
(Slugs)
heterogen
Übergangsbereiche
homogen
2
3
5
8
10
20
30
50
80
100
$d_R$, cm

Fig. 2

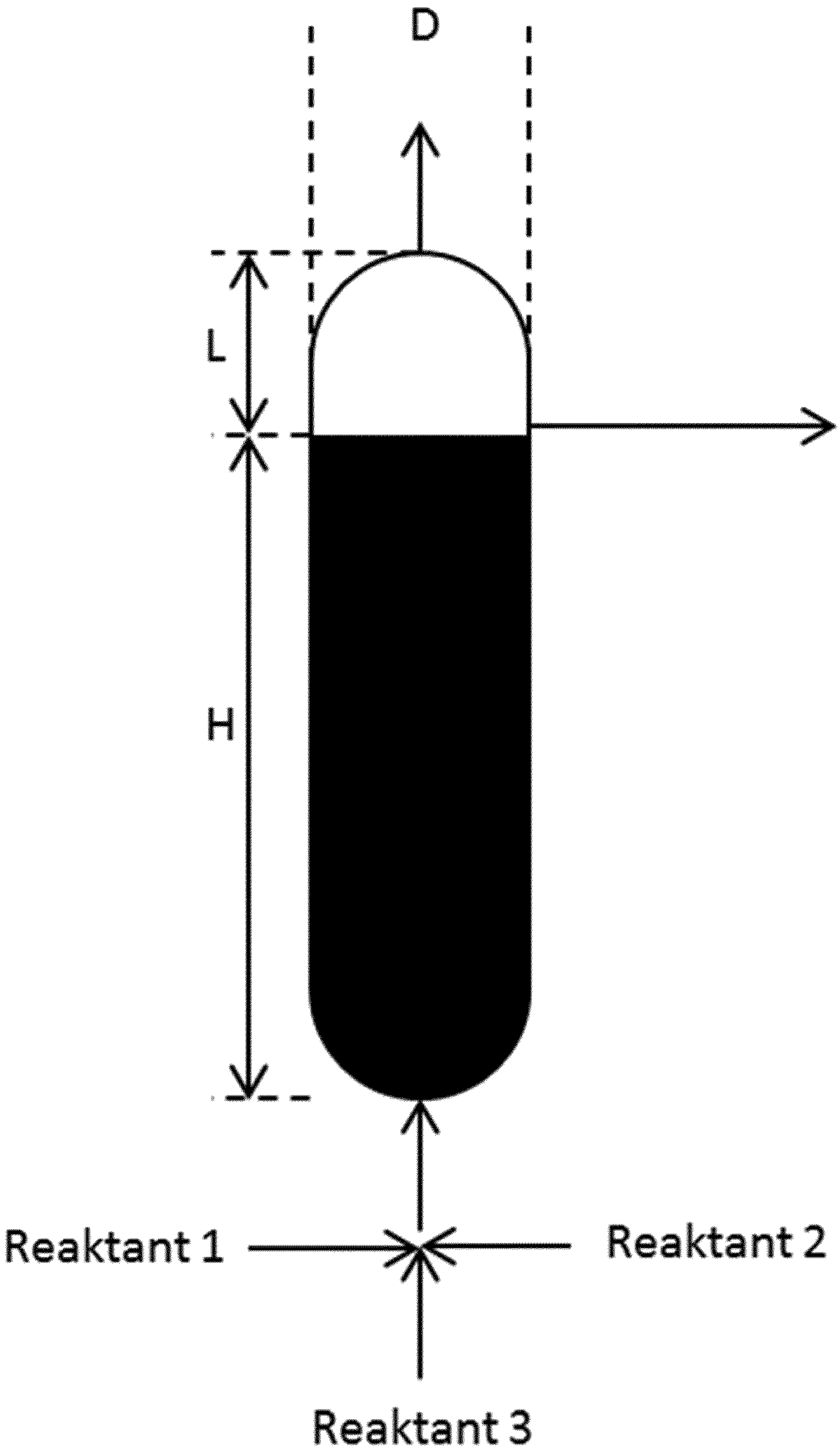


Fig. 3

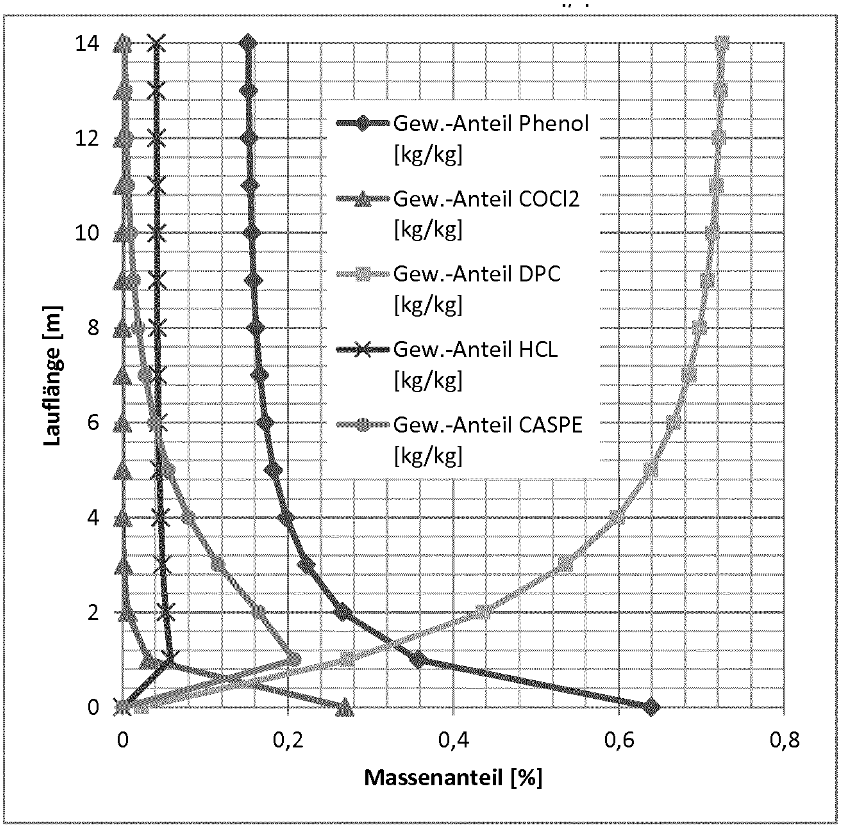
Gew.-Anteil Phenol [kg/kg]
Gew.-Anteil COCl2 [kg/kg]
Gew.-Anteil DPC [kg/kg]
Gew.-Anteil HCL [kg/kg]
Gew.-Anteil CASPE [kg/kg]
Lauflänge [m]
Massenanteil [%]
0
2
4
6
8
10
12
14
0
0,2
0,4
0,6
0,8

Fig. 4

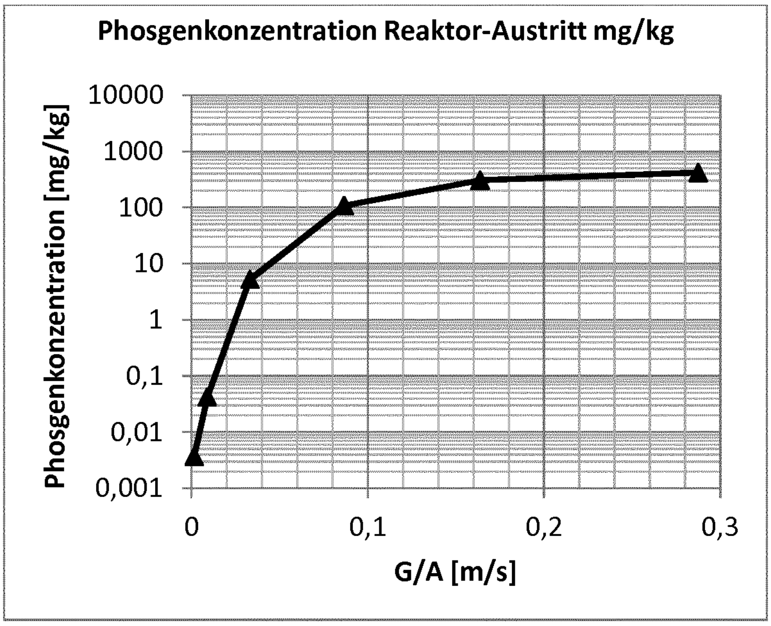
Phosgenkonzentration Reaktor-Austritt mg/kg
Phosgenkonzentration [mg/kg]
G/A [m/s]
10000
1000
100
10
1
0,1
0,01
0,001
0
0,1
0,2
0,3

Fig. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2837555 A
- US 3234263 A
- US 2362865 A
- DE 2447348 C2
- EP 2371806 A1
- DE OS258412 A
- EP 39452 A
- DE OS3445555 A
- JP 54063023 A
- DE OS3445552 A
- DE OS4006520 A
- EP 0338760 A
- JP 57176932 A
- JP 1093580 A
- JP 1093560 A
- JP 61172852 A
- JP 1005588 A
- JP 54125617 A
- DE OS4036594 A
- JP 695219 A
- WO 9106526 A
- US 5424473 A
- EP 516355 A


### In der Beschreibung aufgeführte Nicht-Patentliteratur


- **BLASENSÄULEN.** *Wolf-Dieter Deckwer: Reaktionstechnik in Blasensäulen, Salle + Sauerländer, Frankfurt am Main,* 1985, 184